Europäisches Patentamt

European Patent Office

Office européen des brevets

(19)

(11) Numéro de publication : **0 099 855**
**B1**

# (12) FASCICULE DE BREVET EUROPÉEN

(45) Date de publication du fascicule du brevet :
05.03.86

(21) Numéro de dépôt : **83810264.8**

(22) Date de dépôt : **17.06.83**

(51) Int. Cl.⁴ : **C 07 K   1/06, C 07 K   5/06**

(54) **Procédé de préparation d'un ester dipeptide d'acide L-aspartique.**

(30) Priorité : **07.07.82 CH 4131/82**

(43) Date de publication de la demande :
**01.02.84 Bulletin 84/05**

(45) Mention de la délivrance du brevet :
**05.03.86 Bulletin 86/10**

(84) Etats contractants désignés :
**AT BE CH DE FR GB IT LI LU NL SE**

(56) Documents cités :
**EP-A- 0 027 319**
**FR-A- 2 105 896**
**FR-A- 2 376 127**
**HOUBEN-WEYL: "Methoden der organischen Che-**
**mie", vol. XV/1, "Synthese von Peptiden Teil 1", 1974,**
**pages 46-58, Georg Thieme Verlag, Stuttgart, DE**

(73) Titulaire : **Orgamol S.A.**

**CH-1902 Evionnaz (CH)**

(72) Inventeur : **Baum, Laszlo, Dr.**
**38, Route de Sichoz**
**CH-1814 La Tour-de-Peilz (CH)**
Inventeur : **Szabo, Suzanne**
**1, Avenue de Sully**
**CH-1814 La Tour-de-Peilz (CH)**
Inventeur : **Blanc Gérald**

**CH-1891 Massongex (CH)**

(74) Mandataire : **Micheli, Michel-Pierre**
**MICHELI & CIE 118, Rue du Rhône Case Postale 47**
**CH-1211 Genève 6 (CH)**

## Description

La présente invention a pour objet la préparation de l'ester méthylique d'α-L-aspartyl-L-phénylalanine, par hydrogénation de l'ester méthylique de N-carbobenzoxy-α-L-aspartyl-L-phénylalanine.

Depuis 1932, les carbamates de benzyle ont été largement introduits dans la synthèse des peptides pour le blocage du groupe amino. Ce groupe de protection doit son intérêt à la grande sélectivité qu'il offre vis-à-vis des autres radicaux de blocage, surtout en ce qui concerne son élimination. En effet, l'introduction de ce groupe n'entraîne pas de racémisation, et pour l'éliminer, la réduction catalytique se révèle être une méthode particulièrement intéressante, étant donné que d'autres liaisons (amides, esters, etc...) ne sont pas attaquées par ce moyen de déblocage.

L'emploi industriel de cette méthode, telle que divulguée par exemple dans le document Houben-Weyl « Methoden der Organischen Chemie », vol. XV/1, pages 46-58, Georg Thieme Verlag Stuttgart, 1974, et dans la demande de brevet publiée FR-A-2 105 896, est toutefois limité, car elle nécessite une technique inhabituelle. En effet, au cours de l'hydrogénation se forment du toluène et du $CO_2$, et la réaction ne peut ainsi pas être effectuée en système fermé ; il faut donc opérer dans un courant d'hydrogène, qu'on laisse barboter au fond du réacteur. Cela nécessite en outre un dispositif spécial, pour absorber le $CO_2$ formé, puis pour récupérer l'hydrogène, qu'on utilise en grand excès. De plus, cette méthode requiert l'emploi de solvants tels que des alcools ou, de préférence, l'acide acétique.

Or, lorsque la préparation du produit désiré doit être réalisée à l'échelle industrielle, les caractéristiques du procédé connu mentionnées précédemment deviennent des inconvénients empêchant pratiquement son utilisation dans des conditions satisfaisantes.

En conséquence, le but de la présente invention consiste à fournir un procédé de préparation de l'ester méthylique de l'α-L-aspartyl-L-phénylalanine, utilisable industriellement et dans lequel la réaction de déblocage réducteur est réalisée en l'absence d'hydrogène gazeux.

Le procédé selon l'invention, visant à atteindre le but précité, est caractérisé par le fait qu'on effectue l'hydrogénation par transfert catalytique en utilisant l'acide formique comme donneur d'hydrogène et en présence de palladium comme catalyseur, et par le fait que la réaction est conduite en milieu aqueux.

En effet, la titulaire a constaté de manière surprenante que le déblocage de l'ester méthylique de N-carbobenzoxy-α-L-aspartyl-L-phénylalanine s'effectue pratiquement avec des rendements quantitatifs, et plus rapidement, par hydrogénation par transfert catalytique, en utilisant comme donneur d'hydrogène l'acide formique en présence d'un catalyseur de palladium.

De préférence, on utilise comme catalyseur le palladium sur charbon à 5 %, dans des quantités de 2 à 10 % en poids par rapport à la matière première, et on effectue la réaction sous atmosphère d'azote avec 2 à 10 moles d'acide formique par rapport à 1 mole de N-carbobenzoxy-dérivé.

En général, on mène la réaction à une température se situant entre 20 et 70 °C, pendant 30 minutes à 3 heures.

Selon un mode d'opération particulièrement avantageux, on exécute la réaction dans l'eau, en présence de 4 moles d'acide formique par rapport au produit, à des températures de 40-45 °C. La fin de la réaction est contrôlée par analyse chromatographique en couche mince, qui indique une transformation à 100 % après 2-3 heures ; on filtre alors le catalyseur à chaud, refroidit la solution limpide incolore et ajuste le pH avec $NH_4OH$ à 25 % à 4, 8-5, 3 où l'ester méthylique d'α-L-aspartyl-L-phénylalanine précipite.

La présente invention sera maintenant décrite plus en détail au moyen de l'exemple illustratif suivant :

## Exemple

On prépare une suspension de 85,7 g (0,2 mole) d'ester méthylique de N-carbobenzoxy-L-aspartyl-L-phénylalanine contenant 90 % d'α et 10 % de β-isomère, dans 850 ml d'eau ; on y ajoute 36,8 g (0,8 mole) d'acide formique à 98 %, puis 2,5 g Pd/C à 5 %. Dans un courant d'azote on chauffe le mélange sous agitation lente à 40-45 °C, où on le tient pendant 3 heures, après quoi le contrôle fait par chromatographie couche mince indique que la débenzylation est terminée ; mis à part des isomères α et β du produit final, on remarque des impuretés totales de 2 %. Enfin, on monte la température à 65 °C, filtre le catalyseur, puis cristallise le produit en ramenant le pH entre 4,8 et 5,3 avec environ 27 ml de $NH_4OH$ à 25 %. On brasse le précipité pendant 3 heures entre 0 et 5 °C, puis on filtre et sèche le produit. On obtient ainsi 49,3 g d'ester méthylique d'α-L-aspartyl-L-phénylalanine, correspondant à un rendement de 83,3 %, qui contient 0,4 % de β-isomère.

Ainsi, grâce au procédé selon l'invention, l'ester méthylique de l'α-L-aspartyl-L-phénylalanine peut être obtenu avec de bons rendements sur une échelle industrielle, sans utiliser d'hydrogène gazeux et en travaillant avec l'eau comme solvant, ce qui constitue des avantages particulièrement intéressants par rapport au procédé connu.

## Revendications

1. Procédé de préparation d'ester méthylique de l'α-L-aspartyl-L-phénylalanine par hydrogénation d'ester méthylique de N-carbobenzoxy-L-aspartyl-L-phénylalanine, caractérisé par le fait qu'on effectue l'hydrogénation par transfert catalytique en utilisant l'acide formique comme donneur d'hydrogène et en présence de palladium comme catalyseur, et par le fait que la réaction est conduite en milieu aqueux.

2. Procédé selon la revendication 1, caractérisé par le fait que la réaction est effectuée sous atmosphère inerte.

3. Procédé selon la revendication 1 ou la revendication 2, caractérisé par le fait qu'on emploie comme catalyseur du palladium sur charbon en une quantité de 2 à 10 % en poids par rapport au produit de départ.

4. Procédé selon l'une des revendications 1 à 3, caractérisé par le fait qu'on opère à une température comprise entre 20 et 70 °C.

5. Procédé selon l'une des revendications 1 à 4, caractérisé par le fait que l'on effectue la réaction avec 2 à 10 moles d'acide formique par mole de produit de départ.

6. Procédé selon l'une des revendications 1 à 5, caractérisé par le fait qu'on prépare une suspension dans l'eau de l'ester méthylique de N-carbobenzoxy-L-aspartyl-L-phénylalanine, qu'on ajoute 4 moles d'acide formique par mole de produit de départ à la suspension, qu'on introduit environ 3 % en poids de palladium au charbon 5 %, qu'on chauffe le mélange à 40-45 °C sous atmosphère d'azote, puis qu'on élimine le catalyseur par filtration, qu'on ajuste le pH de la solution entre 4,8 et 5,3, et qu'on cristallise le produit désiré en refroidissant.

## Claims

1. Process for the preparation of methyl ester of α-L-aspartyl-L-phenylalanine by hydrogenating the methyl ester of N-carbobenzoxy-L-phenylalanine, characterized by the fact that the hydrogenation is carried out by catalytic transfer using formic acid as hydrogen donor and in the presence of palladium as catalyst, and by the fact that the reaction is conducted in aqueous medium.

2. Process according to claim 1, characterized by the fact that the reaction is carried out under inert atmosphere.

3. Process according to claim 1 or claim 2, characterized by the fact that palladium-on-charcoal is used as the catalyst in an amount of 2 to 10 wt % relative to the starting product.

4. Process according to one of claims 1 to 3, characterized by the fact that the reaction is carried out at a temperature comprises between 20 and 70 °C.

5. Process according to one of claims 1 to 4, characterized by the fact that the reaction is carried out with 2 to 10 moles of formic acid for one mole of starting product.

6. Process according to one of claims 1 to 5, characterized by the fact that an aqueous suspension in water of the methyl ester of N-carbobenzoxy-L-aspartyl-L-phenylalanine is prepared, that 4 moles of formic acid are added to the suspension for one mole of the starting product, that about 3 wt % of 5 % palladium-on-charcoal are introduced, that the mixture is heated to 40-45 °C under nitrogen atmosphere, and that the catalyst is eliminated by filtration, that the pH is adjusted to between 4,8 and 5,3 and than the aimed product is crystallized by cooling.

## Patentansprüche

1. Verfahren zur Herstellung von α-L-Aspartyl-L-phenylalanin-methylestern durch Hydrierung von N-Carbobenzoxy-L-aspartyl-L-phenylalanin-methylestern, dadurch gekennzeichnet, daß die Hydrierung durch katalytische Übertragung mit Ameisensäure als Wasserstoffdonor und in Gegenwart von Palladium als Katalysator in wäßrigem Medium durchgeführt wird.

2. Verfahren nach Anspruch 1, dadurch gekennzeichnet, daß die Reaktion in inerter Atmosphere durchgeführt wird.

3. Verfahren nach Anspruch 1 oder 2, dadurch gekennzeichnet, daß als Katalysator Palladium auf Kohle in einer Menge von 2 bis 10 Gew.- %, bezogen auf das Ausgangsprodukt, verwendet wird.

4. Verfahren nach einem der Ansprüche 1 bis 3, dadurch gekennzeichnet, daß die Reaktion bei einer Temperatur von 20 bis 70 °C durchgeführt wird.

5. Verfahren nach einem der Ansprüche 1 bis 4, dadurch gekennzeichnet, daß 2 bis 10 mol Ameisensäure je mol Ausgangsprodukt verwendet werden.

6. Verfahren nach einem der Ansprüche 1 bis 5, dadurch gekennzeichnet, daß eine wäßrige Suspension von N-Carbobenzoxy-L-aspartyl-L-phenylalanin-methylester hergestellt wird, die mit 4 mol Ameisensäure je mol Ausgangsprodukt und etwa 3 Gew.- % 5 proz. Palladium auf Kohle versetzt wird, das Gemisch unter Stickstoff auf 40 bis 50 °C erhitzt wird, der Katalysator abfiltriert, der pH-Wert der Suspension auf 4,8 bis 5,3 eingestellt und das gewünschte Produkt durch Abkühlen kristallisiert wird.